# DEMANDE DE BREVET EUROPEEN

(11) **EP 2 075 054 A2**
(43) Date de publication de la demande: **01.07.2009**
(21) Numéro de dépôt: 08291186.8
(22) Date de dépôt: 15.12.2008
(51) Int. Cl.: B01F 17/54, A61K 8/25, A61Q 17/04

(54) **Utilisation d'un polymère organosilicate métallique en tant qu'agent de formation d'une dispersion**

(30) Priorité: 19.12.2007 FR 0708905
(71) Demandeur: Commissariat à l'Energie Atomique, 75015 Paris (FR)
(72) Inventeur: Poncelet, Olivier, 38000 Grenoble (FR); Raccurt, Olivier, 38730 Chelieu (FR); Renard, Olivier, 38120 Fontanil-Cornillon (FR)
(74) Mandataire: Noel, Chantal Odile

(57) **Abrégé**

L'invention concerne l'utilisation d'un polymère organosilicate métallique en tant qu'agent de formation d'une dispersion.

Le polymère organosilicate métallique utilisé dans l'invention à l'une des formules I ou II suivantes :

Formule I : R₄Si₄Al₂O₈(OH)ₓ

Formule II : R₈Si₈M₆O₁₆(OH)_{y}

L'invention trouve application dans le domaine des dispersions de composés hydrophobes dans l'eau et de composés organophobes dans un solvant organique, en particulier.

## Description

L'invention concerne l'utilisation d'un polymère organosilicate métallique en tant qu'agent de formation d'une dispersion d'un matériau hydrophobe dans l'eau ou d'une dispersion d'un matériau organophobe dans un solvant organique.

Elle concerne plus particulièrement une dispersion aqueuse de nanotubes de carbone et une dispersion aqueuse de benzotriazole, ainsi que l'utilisation de la dispersion aqueuse de benzotriazole pour la fabrication d'une composition de protection contre les rayonnements UV.

Il est très difficile de disperser des particules de composés hydrophobes dans une phase aqueuse ou de disperser des particules de composés hydrophiles ou organophobes dans une phase organique.

De nombreux composés organiques sont insolubles dans l'eau.

Il en est ainsi des composés de la famille des benzotriazoles et plus particulièrement du 2-(2'-hydroxy-5'-méthylphényl)benzotriazole.

Or, le 2-(2'-hydroxy-5'-méthylphényl) benzotriazole consomme les photons émis par les UV tout en se régénérant. C'est donc une molécule présentant un grand intérêt pour la protection de divers éléments et supports contre le rayonnement UV car elle n'est pas consommée par irradiation UV.

Mais cette molécule n'est pas dispersable dans l'eau et ne peut pas être appliquée de façon simple et non nocive sur les éléments à protéger.

Les nanotubes de carbone sont, quant à eux, des matériaux inorganiques représentatifs des composés hydrophobes difficiles à disperser dans l'eau.

En général, pour obtenir une dispersion de nanotubes de carbone dans l'eau, on génère des groupements OH à la surface des nanotubes de carbone puis on oxyde ces groupements OH pour former des groupements carboxyliques et enfin on les transforme en sels. Les nanotubes sont alors "dispersables" dans l'eau.

Cependant, cela nécessite de nombreuses réactions chimiques.

Par ailleurs, on connaît une famille de composés appelés indifféremment métal organosilicate, métal phyllosilicate argile, polymère organosilicate métallique ou sel de polysilsesquioxane.

Par exemple, la Demande de brevet US 7 132 165 B2 décrit la fabrication de composés de ce type à partir de surfactants amines ou d'agents de structuration. Ces composés sont décrits comme étant des composés mésoporeux à base de silice lamellaire ayant une stabilité thermique et hydrothermique élevée.

Ces composés sont également décrits par L. Ukrainczyk et al. dans "Template Synthesis and Characterization of Layered AI- and Mg-Silsesquioxanes", J. Phys. Chem. B 1997, 101, 531-539.

Une méthode de préparation de ces composés est décrite dans ce document : les composés silsesquioxanes ont été préparés par précipitation à température ambiante par addition d'une base aqueuse à une solution d'alcool contenant un mélange d'AlCl₃ ou de MgCl₂ et d'un trialcoxysilane avec une fonctionnalité *n*-dodécyle, *n*-octyle, *n*-pentyle, 3-méthacryloxypropyle, isobutyle ou phényle.

Ces composés sont décrits comme utilisables en tant que matériaux absorbants, barrières environnementales, charges de polymères, supports catalytiques ou capteurs chimiques.

Nicola T. Whilton et al., dans "Hybrid lamellar nanocomposites based on organically functionalized magnesium phyllosilicate clays with interlayer reactivity", J. Mater. Chem., 1998, 8(8), 1927-1932, décrivent également la préparation de tels composés par addition d'organotrialcoxysilane à une solution de MgCl₂.6H₂O dans de l'éthanol et précipitation par une solution d'hydroxyde de sodium.

Cependant, dans ce document aucune application n'est décrite ou suggérée pour ces composés.

L'invention vise à fournir des dispersions de composés hydrophobes en phase aqueuse ou de composés organophobes en phase organique.

A cet effet, l'invention propose d'utiliser des particules d'au moins un polymère organosilicate métallique ayant l'une des formules I ou II suivantes :
Formule I : R₄Si₄Al₂O₈(OH)ₓ
Formule II: R₈Si₈M₆O₁₆(OH)_{y}
dans lesquelles :
- chaque R est, indépendamment des autres, un groupement choisi dans le groupe constitué par un groupe alkyle linéaire ou ramifié, substitué ou non substitué, un groupe alcényle linéaire ou ramifié, substitué ou non substitué, un groupe aryle substitué ou non substitué, un groupe benzyle substitué ou non substitué, un groupe vinyle substitué ou non substitué, un groupe polyaromatique substitué ou non substitué et un groupe allyle substitué ou non substitué,
- x≥2.
- y ≥ 2,
- dans la formule I, le rapport molaire Si/Al est compris entre 1,8 et 1,3 inclus,
- dans la formule II, M est choisi dans le groupe constitué par le calcium, le magnésium, le zinc, le strontium et leurs mélanges de deux ou plus, et le rapport molaire Si/M est compris entre 1,8 et 1,3 inclus,
   en tant qu'agent de formation d'une dispersion d'un matériau hydrophobe dans l'eau ou d'une dispersion d'un matériau organophobe dans un solvant organique.

De préférence, dans les formules I et II, chaque R est, indépendamment des autres, choisi parmi un groupe méthyle substitué ou non substitué, un groupe éthyle substitué ou non substitué, un groupe *n*-propyle substitué ou non substitué, un groupe benzyle substitué ou non substitué, et un groupe vinyle substitué ou non substitué.

Dans les formules I et II, lorsque R est substitué, il est substitué par au moins un substituant choisi dans le groupe formé par un groupe amino, un atome d'halogène, un groupe éther, un groupe ester, un groupe hydroxyle, un groupe acrylate, un groupe époxy, un groupe alkyle, un groupe alkylacrylate, un groupe aminoalkyle, un groupe chloroalkyle, un groupe carboxylique, un groupe sulfonique, et un groupe phosphonique.

L'invention propose, dans un mode de réalisation préféré, une dispersion aqueuse de particules d'un matériau hydrophobe, caractérisée en ce qu'elle comprend :
- des particules d'au moins un matériau hydrophobe, et
- des particules d'au moins un polymère organosilicate métallique ayant l'une des formules I et II suivantes :
   Formule I : R₄Si₄Al₂O₈(OH)ₓ,
   Formule II: R₈Si₈M₆O₁₆(OH)_{y}
dans lesquelles :
- chaque R est, indépendamment des autres, un groupement choisi dans le groupe constitué par un groupe alkyle linéaire ou ramifié, substitué ou non substitué, un groupe alcényle linéaire ou ramifié, substitué ou non substitué, un groupe aryle substitué ou non substitué, un groupe benzyle substitué ou non substitué, un groupe vinyle substitué ou non substitué, un groupe polyaromatique substitué ou non substitué et un groupe allyle substitué ou non substitué,
- x ≥ 2,
- y ≥ 4,
- dans la formule I, le rapport molaire Si/Al est compris entre 1, 8 et 1,3 inclus,
- dans la formule II, M est choisi dans le groupe constitué par le calcium, le magnésium, le zinc, le strontium et leurs mélanges de deux ou plus, et le rapport molaire Si/M est compris entre 1,8 et 1,3 inclus.

Dans ce premier mode de réalisation préféré, dans les formules I et II, chaque R est, indépendamment des autres, de préférence choisi parmi un groupe méthyle substitué ou non substitué, un groupe éthyle substitué ou non substitué, un groupe *n*-propyle substitué ou non substitué, un groupe benzyle substitué ou non substitué, ou un groupe vinyle substitué ou non substitué.

Toujours dans ce premier mode de réalisation préféré, lorsque R est substitué, il est de préférence substitué par un groupe formé par un groupe amino, un atome d'halogène, un groupe éther, un groupe ester, un groupe hydroxyle, un groupe acrylate, un groupe époxy, un groupe alkyle, un groupe alkylacrylate, un groupe aminoalkyle, un groupe chloroalkyle, un groupe sulfonique, un groupe carboxylique et un groupe phosphonique.

Dans une première variante du premier mode de réalisation préféré, les particules de matériau hydrophobe sont des nanotubes de carbone.

Dans cette variante, de préférence le polymère organosilicate métallique a la formule II dans laquelle M est le magnésium et quatre des groupes R sont des groupes (CH₂)₃NH(CH₂)₂ et quatre des groupes R sont des groupes C₆H₅.

Toujours dans cette première variante, un autre polymère organosilicate métallique préféré a la formule II dans laquelle M est Mg et quatre des groupes R sont des groupes 3-aminopropyle et quatre des groupes R sont des groupes phényle.

Mais encore un autre polymère organosilicate métallique préféré a la formule II dans laquelle M est Mg et quatre des groupes R sont des groupes 3-aminopropyle et quatre des groupes R sont des groupes vinyle.

Toujours dans cette variante, de préférence, le rapport masse de nanotubes de carbone/masse de polymère organosilicate métallique est supérieur ou égal à 0,5.

Plus préférablement, le rapport masse de nanotubes de carbone/masse de polymère organosilicate métallique est égal à 0,8.

Dans une seconde variante du premier mode de réalisation préféré de l'invention, le matériau hydrophobe est du 2-(2'-hydroxy-5'-méthylphényl)benzotriazole, et il est obtenu en dispersion aqueuse.

Dans ce cas, de préférence, le polymère organosilicate métallique a la formule II dans laquelle M est le zinc et quatre des groupes R sont des groupes (CH₂)₃NH(CH₂)₂ et quatre des groupes R sont des groupes C₆H₅.

L'invention propose également l'utilisation de la dispersion aqueuse selon la seconde variante de l'invention pour la fabrication d'une composition de protection contre les rayonnements UV.

L'invention sera mieux comprise et d'autres caractéristiques et avantages de celle-ci apparaîtront plus clairement à la lecture de la description explicative qui suit.

Les polymères organosilicates métalliques utilisés dans l'invention ont l'une des formules I et II suivantes :
Formule I: R₄Si₄Al₂O₈(OH)ₓ,
Formule II: R₈Si₈M₆O₁₆(OH)_{y}
dans lesquelles :
- chaque R est, indépendamment des autres, un groupement choisi dans le groupe constitué par un groupe alkyle linéaire ou ramifié, substitué ou non substitué, un groupe alcényle linéaire ou ramifié, substitué ou non substitué, un groupe aryle substitué ou non substitué, un groupe benzyle substitué ou non substitué, un groupe polyaromatique substitué ou non substitué, un groupe vinyle substitué ou non substitué, et un groupe allyle substitué ou non substitué,
- x ≥ 2,
- y ≥ 4,
- dans la formule I, le rapport molaire Si/Al est compris entre 1,8 et 1,3 inclus, et
- dans la formule II, M est choisi dans le groupe constitué par le calcium, le magnésium, le zinc, le strontium et leurs mélanges de deux ou plus, et le rapport molaire Si/M est compris entre 1,8 et 1,3 inclus.

Les particules du polymère organosilicate métallique ayant la formule I ou la formule II ci-dessus, ont des tailles de particules comprises entre 10 et 100nm.

Les polymères organosilicates métalliques utilisés dans l'invention sont des composés aluminosilicate, ou silicate d'un métal, comportant des groupes organiques.

Ce sont des composés hygroscopiques. Ils peuvent donc contenir un nombre variable de molécules d'eau, ce qui signifie que le nombre de groupes hydroxyle dans les formules I et II est variable. Cependant, pour respecter la stoechiométrie de ces formules, le nombre de groupes hydroxyle est d'au minimum de 2 dans la formule I et d'au minimum 4 dans la formule II.

Hormis leur caractéristique d'organosilicate, les polymères de formule I utilisés dans l'invention sont caractérisés par un rapport molaire Si/Al compris entre 1,8 et 1,3 inclus, et les polymères de formule II utilisés dans l'invention sont caractérisés par un rapport molaire Si/M compris entre 1,8 et 1,3 inclus.

Ils sont formés par des méthodes de co-hydrolyse contrôlée comprenant le traitement d'un ou plus sel de magnésium, de zinc, de calcium, de strontium ou d'aluminium et d'un mélange d'agents de couplage silane de formule RₓSi(OR¹)₄₋ₓ dans laquelle x vaut entre 1 et 2 inclus et R¹ est un groupe hydrolysable, avec une solution alcaline alcoolique.

Dans certains modes de réalisation, les fonctions R des agents de couplage silane incluent un groupe basique tel qu'un groupe amino et il n'est alors pas nécessaire d'ajouter une solution alcaline alcoolique, bien qu'une petite quantité de base puisse être nécessaire pour faire que la quantité équivalence stoechiométrique désirée de base soit présente. Le rapport molaire métal (un ou plus du magnésium, zinc, calcium, strontium et aluminium)/silicium est de préférence maintenu entre 1 et 0,5 inclus et le rapport molaire alcalin/métal est de préférence maintenu entre 1 et 0,5 inclus.

De préférence, une solution alcoolique d'hydroxyde de sodium, d'hydroxyde de potassium ou d'hydroxyde de lithium de diéthylamine ou de triéthylamine ayant une concentration entre 0,5M et 5M inclus, préférablement d'environ 3M est utilisée.

De préférence, les groupes alcoxy de l'agent de couplage silane (OR¹) sont des groupes propoxy, éthoxy ou méthoxy.

Quant à chaque groupe R présent dans l'agent de couplage silane, il est choisi indépendamment des autres, dans le groupe formé par un groupe alkyle à chaîne linéaire ou ramifiée, substitué ou non substitué, un groupe alcényle linéaire ou ramifié, substitué ou non substitué, un groupe aryle substitué ou non substitué, un groupe benzyle substitué ou non substitué, un groupe vinyle substitué ou non substitué, un groupe polyaromatique substitué ou non substitué, et un groupe allyle substitué ou non substitué.

Le plus préférablement, chaque R est, indépendamment des autres, un groupe alkyle ou un groupe alcényle, chacun linéaire ou ramifié, substitué ou non substitué ou un groupe benzyle substitué ou non substitué ou un groupe vinyle substitué ou non substitué.

Le plus préférablement, chaque R est, indépendamment des autres, un groupe alkyle ou alcényle en C₁ à C₁₈, plus préférablement, en C₂ à C₈ ou un groupe benzyle ou un groupe vinyle, tous ces groupes étant ou non substitués.

Le plus préférablement, chaque R est, indépendamment des autres, un groupe méthyle, un groupe éthyle, un groupe *n*-propyle, un groupe n-butyle, un groupe vinyle ou un groupe benzyle, chacun de ces groupes pouvant être substitué ou non substitué.

Les groupes substituants appropriés du groupe R, et spécialement des groupes alkyles et alcényles linéaires ou ramifiés, peuvent être tout substituant approprié pour affecter les propriétés d'hydrophilie ou d'hydrophobie désirées.

Par exemple, chaque groupe R peut être substitué avec un groupe basique tel qu'un groupe amino, incluant les substituants diamino et triamino, un atome d'halogène, tel qu'un ou plus atome de fluore, de chlore, de brome ou d'iode, mais préférablement un atome de chlore, un groupe éther, un groupe ester, un groupe hydroxyle, un groupe acrylate tel qu'un groupe méthacrylate, ou tout autre groupe partant ou tout autre groupe réactif qui permettra des modifications supplémentaires tel qu'un groupe époxy, ou des groupes aryles optionnellement substitués.

Le plus préférablement, le substituant du groupe R est un groupe alkyle, un groupe alkylacrylate, un groupe aminoalkyle, un groupe chloroalkyle, un groupe sulfonique, un groupe carboxylique ou un groupe phosphonique spécialement un groupe méthyle, un groupe éthyle, un groupe *n*-propyle, un groupe *n*-butyle, un groupe propylméthacrylate, un groupe 3-chloropropyle ou un groupe 3-aminopropyle.

Ainsi, les particules de polymère organosilicate métallique peuvent être fonctionnalisées pour permettre d'obtenir des dispersions d'un matériau hydrophile dans un solvant organique.

Mais les particules de polymère organosilicate métallique pourront être également conçues pour permettre d'obtenir une dispersion aqueuse de particules d'un matériau hydrophobe.

Pour cela, on ajoutera aux particules du matériau hydrophobe des particules d'au moins un polymère organosilicate métallique ayant la formule I ou la formule II telles que définies précédemment.

Grâce à ces particules de polymère organosilicate métallique, on peut obtenir une dispersion aqueuse de nanotubes de carbone par mélange des nanotubes de carbone avec un polymère organosilicate métallique tel que décrit ci-dessus et permettant de disperser des particules d'un matériau hydrophobe.

Dans un premier mode de réalisation préféré, la dispersion aqueuse de nanotubes de carbone comprend des nanotubes de carbone et des particules d'un polymère organosilicate métallique ayant la formule II dans laquelle M est le magnésium et quatre des groupes R sont des groupes (CH₂)₃NH(CH₂)₂ et quatre des groupes R sont des groupes C₆H₅.

Dans un second mode de réalisation préféré, l'invention propose une dispersion aqueuse de nanotubes de carbone comprenant des nanotubes de carbone et des particules d'un composé polymère organosilicate de formule II dans laquelle M est Mg et quatre groupes R sont des groupes 3-aminopropyle et quatre groupes R sont des groupes phényle.

Dans un troisième mode de réalisation préféré, l'invention propose une dispersion aqueuse dans laquelle le polymère organosilicate métallique a la formule II dans laquelle M est Mg et quatre des groupes R sont des groupes 3-aminopropyle et quatre des groupes R sont des groupes vinyle.

Dans ces trois modes de réalisation préférés, pour obtenir la stabilisation de la dispersion aqueuse de nanotubes de carbone pendant au moins une heure, le rapport masse de nanotubes/masse de polymère organosilicate métallique est, de préférence, supérieur ou égal à 0,5.

Pour obtenir une dispersion aqueuse de nanotubes de carbone stable pendant au moins trois heures, le rapport masse de nanotubes de carbone/masse de particules de polymère organosilicate métallique est de 0,8.

Dans une seconde variante, l'invention propose une dispersion aqueuse de composés benzotriazole et, plus particulièrement, de 2-(2'-hydroxy-5'-méthylphényl)benzotriazole.

Dans cette variante, de préférence les particules de polymère organosilicate métallique sont des particules d'un polymère organosilicate métallique de formule II dans laquelle M est le zinc et quatre des groupes R sont des groupes (CH₂)₃NH(CH₂)₂ et quatre des groupes R sont des groupes C₆H₅.

Cette dispersion aqueuse obtenue pourra être utilisée, en particulier pour la fabrication d'une composition de protection contre les rayonnements UV, en particulier lorsqu'elle est appliquée en film mince.

En effet, une irradiation UV rompt la liaison hydrogène qui permet au 2-(2'-hydroxy-5'-méthylphényl)benzotriazole d'être aromatique, ce qui fait que cette molécule absorbe les photons UV.

Comme avec le temps une nouvelle liaison hydrogène se forme, cela fait que cette molécule est un "absorbeur" de photons UV régénérable.

Mais cette molécule, bien qu'agissant en tant que filtre des UV, est très sensible à l'oxydation.

Or, on a constaté que l'ajout d'un polymère organosilicate métallique selon l'invention, non seulement permet de disperser cette molécule dans l'eau, mais également de la protéger contre l'oxydation.

Afin de mieux faire comprendre l'invention, on va maintenant décrire à titre d'exemples purement illustratifs et non limitatifs plusieurs modes de mise en oeuvre.

### Exemple 1 : Synthèse de particules de polymère organosilicate métallique de formule II dans laquelle M est Mg et quatre groupes R sont des groupes 3-aminopropyle et quatre groupes R sont des groupes phényle.

On solubilise 0,0191 mole de MgCl2.6H2O dans 200g d'éthanol, puis un mélange équimolaire de (3-aminopropyl)triéthoxysilane et de phényltriéthoxysilane (0,00955/0,00955) dans 20g d'EtOH est rapidement ajouté. Rapidement la solution s'opacifie, on ajoute alors 0,0095 mole de NaOH dans 20g d'EtOH. Le milieu réactionnel est agité à température ambiante durant 24H. Après filtration, le précipité blanc est lavé avec 200g d'éthanol. La poudre blanche obtenue est séchée à température ambiante pendant deux jours. Le rendement calculé sur le magnésium est de 49%. Le rapport molaire Mg/Si mesuré par ICP-AES (Inductively Coupled Plasma Atomic Emission Spectrophotometry) est de 0,7. La diffraction des rayons X montre que le produit est faiblement cristallisé. Le polymère organosilicate métallique obtenu est entièrement dispersable dans l'eau, il peut être lavé par dialyse ou par nano et/ou ultrafiltration. La taille des particules mesurée par spectroscopie de corrélation de photons se situe entre 20 et 50nm. Les mesures de taille des particules sont effectuées à des concentrations en polymère comprises entre 5 et 30g/l. On ne note pas d'accroissement de la taille des particules dans ce domaine de concentrations.

### Exemple 2 : Synthèse de particules d'un polymère organosilicate métallique de formule II dans laquelle M est Mg et quatre des groupes R sont des groupes 3-aminopropyle et quatre des groupes R sont des groupes vinyle.

On solubilise 0,0191 mole de MgCl₂.6H₂O dans 200g d'éthanol, puis un mélange équimolaire de (3-aminopropyl)triéthoxysilane et de vinyltriméthoxysilane (0,00955/0,00955) dans 20g d'EtOH est rapidement ajouté. Rapidement la solution s'opacifie, on ajoute alors 0,0095 mole de NaOH dans 20g d'EtOH. Le milieu réactionnel est agité à température ambiante durant 24H. Après filtration, le précipité blanc est lavé avec 200g d'éthanol. La poudre blanche obtenue est séchée à température ambiante pendant deux jours. Le rendement calculé sur le magnésium est de 48%. Le rapport molaire Mg/Si mesuré par ICP-AES (Inductively Coupled Plasma Atomic Emission Spectrophotometry) est de 0,65. La diffraction des rayons X montre que le produit est faiblement cristallisé. Le polymère organosilicate métallique obtenu est entièrement dispersable dans l'eau, il peut être lavé par dialyse ou par nano et/ou ultrafiltration. La taille des particules mesurée par spectroscopie de corrélation de photons se situe entre 20 et 50nm. Les mesures de taille des particules sont effectuées à des concentrations en polymère organosilicate métallique comprises entre 5 et 30g/l. On ne note pas d'accroissement de la taille des particules dans ce domaine de concentrations.

Dans les exemples qui suivent, l'efficacité des particules de polymère organosilicate métallique selon l'invention pour obtenir des dispersions aqueuses de composés hydrophobes et en particulier de nanotubes de carbone et de 2-(2'-hydroxy-5'-méthylphényl)benzotriazole a été évaluée.

### Exemple 3 : Synthèse de particules d'un polymère organosillicate métallique dans lequel M est Mg et quatre groupes R sont des groupes 3-aminopropyle et quatre groupes R sont des groupes CH₃(CH₂)₁₇·

9,75g de MgCl₂, 6H₂O sont solubilisés dans 160ml d'EtOH puis on ajoute un mélange équimolaire (0,03 mole de CH₃(CH₂)₁₇Si(OEt)₃ +0,03 mole de NH₂(CH₂)3Si(OEt)₃ dans 20ml d'EtOH sous agitation à température ambiante. La solution devient légèrement turbide. On ajoute alors 0,03 mole de KOH dans 20 ml d'EtOH (KOH peut être remplacé par du NaOH ou du LiOH ou une base organique triméthylamine, cependant les bases inorganiques sont préférées). Un précipité blanc se forme. Le milieu réactionnel est laissé sous agitation à température ambiante pendant 12H, puis filtré sur Büchner. La poudre blanche obtenue est lavée avec 150 ml d'EtOH puis séchée à température ambiante pendant deux jours. Le rendement calculé sur le magnésium est de 47%. Le rapport molaire Mg/Si mesuré par ICP-AES (Inductively Coupled Plasma Atomic Emission Spectrophotometry) est de 0,7. La diffraction des rayons X montre que le produit est faiblement cristallisé. Le polymère organosillicate métallique obtenu est entièrement dispersable dans l'eau, il peut être lavé par dialyse ou par nano et/ou ultrafiltration. La taille des particules mesurée par spectroscopie de corrélation de photons se situe entre 20 et 60nm dans l'eau. Les mesures de taille des particules sont effectuées à des concentrations en polymère organosilicate métallique comprises entre 5 et 20g/l. On ne note pas d'accroissement de la taille des particules dans ce domaine de concentrations.

### Exemple 4 : Dispersion aqueuse de nanotubes de carbone.

Les nanotubes de carbone utilisés ont été fournis par la société ARKEMA.

Le protocole d'évaluation est le suivant : une quantité connue de nanotubes est mélangée à de l'eau osmosée contenant ou non un polymère organosilicate métallique, les récipients sont mis à agiter dans un mélangeur orbital de marque Turbula^{®} (système Schatz) fabriqué par W.A. Bachofen.AG à température ambiante. Les récipients sont ensuite mis au repos pendant une heure, la transmission de la lumière visible du milieu surnageant est ensuite mesurée dans des cellules quartz de 1 mm de trajet optique. 0% de transmission signifie que rien ne passe à travers et que donc les nanotubes de carbone sont en suspension.

On prépare plusieurs échantillons :
Echantillon 4A : 50 mg de nanotubes de carbone ARKEMA dans 2ml d'eau osmosée.
Echantillon 4B : 50 mg de nanotubes de carbone ARKEMA dans 2ml d'une solution à (30g/l) en NaCl.
Echantillon 4C : 50 mg de nanotubes de carbone ARKEMA dans 2ml d'un sol colloïdal aqueux du polymère organosilicate métallique obtenu à l'exemple 3 (20g/l).
Echantillon 4D : 50 mg de nanotubes de carbone ARKEMA dans 2ml d'un sol colloïdal aqueux du polymère organosilicate métallique obtenu à l'exemple 1 (30g/l).
Echantillon 4E : 50 mg de nanotubes de carbone ARKEMA dans 2ml d'un sol colloïdal aqueux du polymère organosilicate métallique obtenu à l'exemple 2 (30g/l).

Tous les milieux réactionnels étudiés avant d'être mis en contact avec les nanotubes de carbone sont parfaitement transparents en lumière visible, leur transmission est donc de 100%.

Les résultats de transmission de la lumière obtenus après mise en contact avec les nanotubes de carbone sont donnés dans le tableau 1 ci-après:

**Tableau 1**

| Echantillons | % lumière transmise après une heure de décantation | % lumière transmise après 3 heures de décantation |
|---|---|---|
| Echantillon 4A | 100 | 100 |
| Echantillon 4B | 97 | 98 |
| Echantillon 4C | 0 | 10 |
| Echantillon 4D | 0 | 0 |
| Echantillon 4E | 0 | 0 |

Même dans une solution concentrée en sel (exemple 4B), la suspension de nanotubes de carbone n'est pas stable, elle finit par décanter. Par contre, la présence de polymère organosilicate métallique permet de stabiliser la suspension formant une encre noire ne laissant pas passer la lumière. Après une heure, les dispersions ou suspensions contenant un polymère organosilicate métallique selon l'invention sont toujours stables.

Après trois heures, les dispersions ou suspensions dans lesquelles le polymère organosilicate métallique porte des groupements organiques insaturés sont toujours stabilisées.

Le rapport massique nanotubes de carbone/polymère organosilicate métallique est de préférence de 0,8.

Il est également de préférence supérieur à 0,5 car en dessous de 0,5, à l'oeil, il semble moins facile de disperser les nanotubes de carbone.

### Exemple 5: Synthèse de particules d'un polymère organosilicate métallique de formule II dans laquelle M est Zn et quatre des groupes R sont des groupes (CH₂)₃NH(CH₂)₂ et quatre groupes R sont des groupes benzyle (C₆H₅).

1,2 moles de ZnCl₂.6H₂O sont solubilisés dans 2900g d'éthanol, 312g de (3-aminopropyl)triéthoxysilane sont rapidement ajoutés. Rapidement un précipité blanc se forme, le milieu réactionnel est agité à température ambiante durant 24H. Après filtration, le précipité blanc est lavé avec 2000g d'éthanol. La poudre blanche obtenue est séchée à température ambiante pendant deux jours. Le rendement calculé sur le magnésium est de 48%. Le rapport molaire Zn/Si mesuré par ICP-AES (Inductively Coupled Plasma Atomic Emission Spectrophotometry) est de 0,57. La diffraction des rayons X montre que le produit est faiblement cristallisé. Le composé polymère organosilicate métallique obtenu est entièrement dispersable dans l'eau, il peut être lavé par dialyse ou par nano et/ou ultrafiltration. La taille des particules mesurée par spectroscopie de corrélation de photons se situe entre 20 et 30nm. Les mesures de taille des particules sont effectuées à des concentrations en polymère organosilicate métallique comprises entre 5 et 35g/l. On ne note pas d'accroissement de la taille des particules dans ce domaine de concentrations.

### Exemple 6 : Dispersion aqueuse de 2-(2'-hydroxy-5'-méthylphényl)benzotriazole.

25ml d'un sol colloïdal aqueux du polymère organosilicate métallique obtenu à l'exemple 5 (10g/l) sont mis sous agitation à température ambiante, on ajoute ensuite 200 mg de 2-(2'-hydroxy-5'-méthylphényl) benzotriazole. Celui-ci est tout à fait insoluble dans l'eau. La dispersion dans le sol colloïdal est très lente, elle est accélérée quand on irradie le dessus du Bécher avec une lampe UV (4W centrée à 365nm).

La dispersion devient limpide et transparente en quelques minutes. Sans cette irradiation, la dispersion est très lente voire impossible. L'irradiation UV rompt la liaison hydrogène qui confère la planéité donc la délocalisation des électrons et par là-même son absorbance dans les UV au 2-(2'-hydroxy-5'-méthylphényl)benzotriazole, celui-ci se dépolymérise et est rapidement adsorbé par le polymère organosilicate métallique.

La dispersion obtenue est stable dans le temps.

### Exemple 7 : Utilisation de la dispersion aqueuse de 2-(2'-hydroxy-5'-methylphényl)benzotriazole pour la fabrication d'une composition de protection contre les UV

Le 2-(2'-hydroxy-5'-methylphényl)benzotriazole est un composé absorbant les photons UV et donc un excellent agent de protection contre les UV. Grâce à l'utilisation d'un polymère organosilicate métallique selon l'invention, ce composé peut être utilisé en dispersion aqueuse pour la fabrication de couches minces. Mais il est sensible à l'oxydation. Le but de cet exemple est de montrer que la dispersion aqueuse de ce composé avec un polymère organosilicate métallique est stabilisée vis-à-vis de l'oxydation et qu'ainsi, cette dispersion peut être utilisée pour la fabrication d'une composition de protection contre les UV.

Afin de mesurer la stabilisation du 2-(2'-hydroxy-5'-méthylphényl)benzotriazole à l'oxydation, on fabrique des couches minces de compositions suivantes :
- Composition 10A : on introduit 5ml de la dispersion de 2-(2'-hydroxy-5'-méthylphényl)benzotriazole obtenue précédemment dans une formulation sol-gel faite d'un mélange alcoolique 0,1M de quatre équivalents molaires de 3-glycidopropyltriméthoxysilane + 1 équivalent molaire de tétraéthyle orthosilicate + 5ml d'H₂O.
   On a ainsi substitué les 5ml d'H₂O de la formulation sol-gel du mélange alcoolique 0,1M par 5ml de la dispersion de 2-(2'-hydroxy-5'-méthylphényl)benzotriazole obtenue à cet exemple.
- Composition 10B : on introduit 0,5ml de la dispersion de 2-(2'-hydroxy-5'-méthylphényl)benzotriazole obtenue à cet exemple dans la formulation sol-gel du mélange alcoolique 0,1 M.

Dans ce cas, seulement 10% des 5ml d'H₂O de cette formulation sol-gel sont remplacés par la dispersion aqueuse de 2-(2'-hydroxy-5'-méthylphényl)benzotriazole obtenue à cet exemple.

Composition 10C: A titre comparatif, on prépare une composition contenant 100mg de 2-(2'-hydroxy-5'-méthylphényl)benzotriazole non stabilisés par un polymère organosilicate métallique selon l'invention dilué dans de l'alcool polyvinylique (4%), du THF (25ml), et de l'eau (10ml).

Ces compositions sont déposées sur des lames de verre et séchées.

On obtient alors trois couches minces notées respectivement 10A, 10B et 10C.

Toutes les couches obtenues sont parfaitement transparentes et homogènes.

Toutes les couches obtenues filtrent les UV dans la région de 280 à 380nm. Le pourcentage absorbé dans le domaine UV est directement proportionnel à la quantité de 2-(2'-hydroxy-5'-méthylphényl)benzotriazole présent.

Les transmittances de ces couches minces transparentes sont mesurées :
- dès leur fabrication,
- après 48 heures à l'abri de la lumière sous hôte aspirante (10m³/h),
- après 24 heures sous irradiation UV (4W centrée à 365nm) à l'air, et
- après 24 heures sous irradiation UV (4W centrée à 365nm) sous argon.

Les résultats obtenus sont reportés au tableau 2 suivant :

**Tableau 2**

| Echantillons | % transmittance dès fabrication | % transmittance après 48 heures à l'abri de la lumière | % transmittance après 24 heures d'exposition aux UV à l'air | % transmittance après 24 heures d'exposition aux UV sous argon |
|---|---|---|---|---|
| Couche mince 10A | 85% | 85% | 85% | 84% |
| Couche mince 10B | 10% | 10% | 10% | 11% |
| Couche mince 10C | 87% | 65% | 60% | 60% |

Ces résultats confirment que le polymère organosilicate métallique de l'invention stabilise le 2-(2'-hydroxy-5'-méthylphényl)benzotriazole contre l'oxydation.

## Revendications

1. Utilisation de particules d'au moins un polymère organosilicate métallique ayant l'une des formules I ou II suivantes :
Formule I: R₄Si₄Al₂O₈(OH)ₓ,
Formule II : R₈Si₈M₆O₁₆(OH)_{y}
dans lesquelles :
- chaque R est, indépendamment des autres, un groupement choisi dans le groupe constitué par un groupe alkyle linéaire ou ramifié, substitué ou non substitué, un groupe alcényle linéaire ou ramifié, substitué ou non substitué, un groupe aryle substitué ou non substitué, un groupe benzyle substitué ou non substitué, un groupe vinyle, substitué ou non substitué, un groupe polyaromatique substitué ou non substitué et un groupe allyle substitué ou non substitué,
- x ≥ 2,
- y ≥ 4,
- dans la formule I, le rapport molaire Si/Al est compris entre 1,8 et 1,3 inclus,
- dans la formule II, M est choisi dans le groupe constitué par le calcium, le magnésium, le zinc, le strontium et leurs mélanges de deux ou plus, et le rapport molaire Si/M est compris entre 1,8 et 1,3 inclus,
en tant qu'agent de formation d'une dispersion d'un matériau hydrophobe dans l'eau ou d'une dispersion d'un matériau organophobe dans un solvant organique.

2. Utilisation selon la revendication 1, **caractérisée en ce que** dans les formules I et II chaque R est, indépendamment des autres, choisi parmi un groupe méthyle substitué ou non substitué, un groupe éthyle substitué ou non substitué, un groupe *n*-propyle substitué ou non substitué, un groupe benyzle substitué ou non substitué et un groupe vinyle substitué ou non substitué.

3. Utilisation selon la revendication 1 ou 2, **caractérisée en ce que** dans les formules I et II, lorsque R est substitué, il est substitué par au moins un substituant choisi dans le groupe formé par un groupe amino, un atome d'halogène, un groupe éther, un groupe ester, un groupe hydroxyle, un groupe acrylate, un groupe époxy, un groupe alkyle, un groupe alkylacrylate, un groupe aminoalkyle, un groupe chloroalkyle, un groupe carboxylique, un groupe phosphonique et un groupe sulfonique.

4. Dispersion aqueuse de particules d'un matériau hydrophobe, **caractérisée en ce qu'**elle comprend :
- des particules d'au moins un matériau hydrophobe, et
- des particules d'au moins un polymère organosilicate métallique ayant l'une des formules I ou II suivantes :
Formule I: R₄si₄Al₂O₈(OH)ₓ,
Formule II : R₈Si₈M₆O₁₆(OH)_{y}
dans lesquelles :
- chaque R est, indépendamment des autres, un groupement choisi dans le groupe constitué par un groupe alkyle linéaire ou ramifié, substitué ou non substitué, un groupe alcényle linéaire ou ramifié, substitué ou non substitué, un groupe aryle substitué ou non substitué, un groupe benzyle substitué ou non substitué, un groupe vinyle substitué ou non substitué, un groupe polyaromatique substitué ou non substitué et un groupe allyle substitué ou non substitué,
- dans la formule I, le rapport molaire Si/Al est compris entre 1,8 et 1,3 inclus,
- dans la formule II, M est choisi dans le groupe constitué par le calcium, le magnésium, le zinc, le strontium et leurs mélanges de deux ou plus, et le rapport molaire Si/M est compris entre 1,8 et 1,3 inclus.

5. Dispersion selon la revendication 4, **caractérisée en ce que** dans les formules I et II chaque R est, indépendamment des autres, choisi parmi un groupe méthyle substitué ou non substitué, un groupe éthyle substitué ou non substitué, un groupe *n*-propyle substitué ou non substitué, un groupe benzyle substitué ou non substitué, et un groupe vinyle substitué ou non substitué.

6. Dispersion selon la revendication 4 ou 5, **caractérisée en ce que** dans les formules I et II, lorsque R est substitué, il est substitué par au moins un substituant choisi dans le groupe formé par un groupe amino, un atome d'halogène, un groupe éther, un groupe ester, un groupe hydroxyle, un groupe acrylate, un groupe époxy, un groupe alkyle, un groupe alkylacrylate, un groupe aminoalkyle, un groupe chloroalkyle, un groupe carboxylique, un groupe sulfonique et un groupe phosphonique.

7. Dispersion selon l'une quelconque des revendications 4 à 6, **caractérisée en ce que** les particules de matériau hydrophobe sont des nanotubes de carbone.

8. Dispersion selon la revendication 7, **caractérisée en ce que** le polymère organosilicate métallique a la formule II dans laquelle M est le magnésium et quatre groupes R sont des groupes (CH₂)₃NH(CH₂)₂ et quatre groupes R sont des groupes C₆H₅.

9. Dispersion selon la revendication 7, **caractérisée en ce que** le polymère organosilicate métallique a la formule II dans laquelle M est Mg et quatre groupes R sont des groupes 3-aminopropyle et quatre groupes R sont des groupes phényle.

10. Dispersion aqueuse selon la revendication 7, **caractérisée en ce que** le polymère organosilicate métallique a la formule II dans laquelle M est Mg et quatre des groupes R sont des groupes 3-aminopropyle et quatre des groupes R sont des groupes vinyle.

11. Dispersion selon l'une quelconque des revendications 7 à 9, **caractérisée en ce que** le rapport masse de nanotubes de carbone/masse de polymère organosilicate métallique est supérieur ou égal à 0,5.

12. Dispersion selon l'une quelconque des revendications 7 à 10, **caractérisée en ce que** le rapport masse de nanotubes de carbone/masse de polymère organosilicate métallique est égal à 0,8.

13. Dispersion aqueuse selon l'une quelconque des revendications 4 à 6, **caractérisée en ce que** le matériau hydrophobe est le 2-(2'-hydroxy-5'-méthylphényl)benzotriazole.

14. Dispersion aqueuse selon la revendication 13, **caractérisée en ce que** le polymère organosilicate métallique a la formule II dans laquelle M est le zinc et quatre groupes R sont des groupes (CH₂)₃NH(CH₂)₂ et quatre groupes R sont des groupes C₆H₅.

15. Utilisation de la dispersion aqueuse selon la revendication 13 ou la revendication 14 pour la fabrication d'une composition de protection contre les rayonnements UV.
